# EUROPEAN PATENT SPECIFICATION

(11) **EP 0 745 843 B1**
(45) Date of publication and mention of the grant of the patent: **28.07.1999**
(21) Application number: 96303979.7
(22) Date of filing: 31.05.1996
(51) Int. Cl.: G01N 27/327, G01N 33/543, G01N 33/58

(54) **Electrochemical immunobiosensor**
Elektrochemischer Immunobiosensor
Immunobiocapteur électrochimique

(30) Priority: 01.06.1995 KR 1452295
(43) Date of publication of application: 04.12.1996
(73) Proprietor: LG ELECTRONICS INC., Seoul (KR)
(72) Inventor: Park, Je-Kyun, Seoul (KR); Yee, Hee-Jin, Seoul (KR)
(74) Representative: Cross, Rupert Edward Blount

(56) References cited:
- EP-A- 0 352 138
- WO-A-91/16630
- DE-A- 3 618 101
- ANAL. CHEM. (1980), 52(11), 1610-13 CODEN: ANCHAM;ISSN: 0003-2700, 1980, XP000615736 SHIBA, KUNIO ET AL: "Thin-layer potentiometric analysis of lipid antigen-antibody reaction by tetrapentylammonium (TPA+) ion loaded liposomes and TPA+ ion selective electrode"
- ANAL. CHEM. (1988), 60(2), 142-7 CODEN: ANCHAM;ISSN: 0003-2700, 1988, XP000615737 KANNUCK, ROSANNE M. ET AL: "Measurement of liposome - released ferrocyanide by a dual-function polymer modified electrode"
- BIOTECHNOL. TECH. (1995), 9(6), 389-94 CODEN: BTECE6;ISSN: 0951-208X, 1995, XP000614545 KIM, H.-J. ET AL: "A novel liposome-based electrochemical biosensor for the detection of hemolytic microorganisms"

## Description

### FIELD OF THE INVENTION

The present invention relates to an electrochemical immunobiosensor. More specifically, the present invention relates to an immunobiosensor which can quantitatively analyse the concentration of physiolocially important materials present in a sample by electrochemically measuring the result of an immunochemical reaction between an antigen and an antibody.

### BACKGROUND OF THE INVENTION

As the reaction occurs with high specificity and strong binding force in an antigen-antibody reaction, the immunobiosensor according to the invention has more favourable characteristics than previous sensors used in assaying the physiologically important materials. Conventional immunobiosensors generally use optical phenomenon. A method for measuring the light intensity which alters with the result of interaction between an immobilized antibody at the end of an optical fiber and an analyte (antigen) has been reported [Anderson G.P. et al., IEEE Trans. Biomed. Eng. 1994, 41 578-584]. A further method comprises fixing an antibody on a piezoelectric element and altering the change of mass, induced by binding thereof with an analyte (antigen), into an electric sing [König. B. et al., Anal. Chem. 1994, 66, 341-344]. A method detecting the change of surface plasom resonance phenomenon occuring at the liquid interface with a metal by binding of an immobilized antibody on the metal with an analyte (antigen) [Severs, A.M. et al., Biosensors & Bioelectronics, 1993, 8, 365-370] or the like has also been reported.

As an immunobiosensor using an electrochemical method, a method which comprises generating electroactive species by using conventional immunoassay such as enzyme-linked immunoSorbent assay(ELISA) or metal ion labelling, separating thereof to measure electrochemically has also been reported [Hayes, F.J. et al., Anal. Chem. 1994, 66, 1860-1865].

Liposome immunoassay methods in which a labelled material is encapsulated by the liposome and the liposome is ruptured after the completion of the immunochemical reaction to release the labelled material so that a signal related to the concentration of the analyte is provided, are recently in the spotlight. Liposomes have been investigated as a drug delivery system because their main component is phospholipid and they have the property of being able to capture a hydrophilic or hydrophobic material or to adjust the passage of the entrapped material, dependent upon the characteristics of the bilayer, as the properties of the cell membrane. A variety of experiments for applying these liposomes in immunoassays have been performed recently, however, few examples have been reported applying liposomes in a sensor [Richard, A. et al., Biosensors & Bioelectronics, 1993, 8, xiii-xv].

Conventional immunobiosensors using optical phenomenon generally have the disadvantage that the measuring system is complicated and costly. In contrast, the immunobiosensors to which the principles of electrochemistry have been applied have a very uncomplicated measuring system, and the process for production of electrodes is also relatively easy.

Though a conventional immunoassay using electrochemical measuring system has been reported [Athey, D. et al., Biosensors & Bioelectronics, 1993, 8, 415-419], it generally takes a long time for the assay to be performed because the procedure uses a conventional immunoassay system and electrochemical measurement is only used in the final assay stage, therefore the assay is not easy in practical terms because a variety of complicated stages occur during the assay. Generally, an immunochemical reaction such as an antigen-antibody reaction requires a longer time period because of the characteristics of the reaction per se.

Electrochemical immunobiosensors generally remain undeveloped because of the technical difficulties in inducing an electroactive species from an antigen-antibody complex. In the case of an electrochemical sensor using an enzyme, electroactive species are readily formed on an electrode as a result of the enzyme reaction on the electrode and the material is oxidized or reduced to produce an electrical current. However, when an antibody is reacted with an analyte (antigen) on an electrode, no electroactive species is produced. In order to alleviate such difficulties, attempts for preparation of an antibody having enzyme activity have been reported.

An immunoassay using a liposome and its application as a sensor is also complicated, because generally in an immunoassay, the required material for each step should be sequentially added, while in the case of recently introduced immunobiosensors which utilise liposomes, liposomes as well as a sample should be added so that the user must treat additional material containing liposomes as well as the sensor itself at the time of using thereof. Besides, liposomes must be ruptured by adding additional material in order to perform an electrochemical measurement, whereby the method is not simple.

An immunobiosensor using an immunochemical reaction such as an antigen-antibody reaction cannot be produced in a strip type sensor such as that of conventional thick-film enzyme sensors. This is because no electroactive species occur as a result of the immunochemical reaction itself; and in case of so called "sandwich assay" [where the antibody is fixed on the upper part of the electrode and then analyte (antigen) in the sample is bound to the antibody, and it is reacted with the second antibody combined to an enzyme or electroactive species], the sensor must be washed during the assay and auxiliary reagents are needed.

### SUMMARY OF THE INVENTION

It would therefore be desirable to overcome the above mentioned problems and to provide an electrochemical immunobiosensor which can directly measure electrochemically the result of antigen-antibody reaction, by taking advantage of liposomes which can comprise excess amount of electroactive species as a signal-generating source, and the principles of electrochemistry having a simple measuring system.

It would be desirable to provide a disposable strip type immunobiosensor which can perform an assay in a relatively short time by dropping a sample onto the sensor, which obviates a complicated multi-step operation during the assay.

It would also be desirable to provide a disposable immunobiosensor having a structure wherein a chromatographic matrix (which causes immunochemical reaction by developing the sample) is combined with a strip type electrode, in order to quantify the concentration of the material to be assayed in the sample by contacting and reacting the sensor with the sample once without any additional auxiliary reagent.

It would also be desirable to provide an electrochemical immunobiosensor in which an immunochemical reaction occurs while the sample is developed along the chromatographic matrix, having a structure wherein an analyte-cytolytic agent conjugates, an antibody(or antigen) which selectively combines with the analyte, and liposomes containing electroactive species are adsorbed or immobilized in a specific site respectively.

It would also be desirable to provide an electrochemical immunobiosensor which can accurately measure the physiologically important analytes at a high sensitivity by integrating the matrix and the electrode prepared on the insulating base plate so that the electroactive species released by the liposomelysis can be effectively oxidized or reduced by using the principles of generation of signal from electroactive species-containing liposomes.

It would also be desirable to provide a multi-immunobiosensor for measuring multicomponents.

An electrochemical immunobiosensor satisfying the desires described above may comprise an electrode part where a plurality of electrodes are formed on an insulating base plate and a part of the electrode is exposed by selectively forming an insulating layer on the electrode; and a matrix part where a portion absorbed with analyte-cytolytic agent conjugates, a portion immobilized with a recepter which binds with the analyte, and a portion immobilized with electroactive species-containing liposomes, to perform the functions of liposome-based amplification signal and immunochromatography; and said electrode portion exposed in the electrode part and the liposome portion in the matrix are integrated.

In a currently preferred embodiment the multi-immunobiosensor comprises overlapping a plurality of the electochemical immunobiosensors which comprises an electrode part where a plurality of electrodes are formed on an insulating base plate and a part of the electrode is exposed by selectively forming an insulating layer on the electrode; and a matrix part where a portion absorbed with analyte-cytolytic agent conjugates, a portion immobilized with a recepter which binds with the analyte, and a portion immobilized with electroactive species-containing liposomes; and said electrode portion exposed in the electrode part and the liposome portion in the matrix are integrated, with comparative sensor which generates reference signal to enable simultaneous measurement of physiologically important multi-component species.

### BRIEF DESCRIPTION OF THE DRAWINGS

FIG. 1 is a perspective view of a 3-electrode system of an immunobiosensor embodying the present invention.

FIG. 2 is a plane view of a 3-electrode system of an immunobiosensor embodying the present invention.

FIG. 3 is a perspective view of a 2-electrode system of an immunobiosensor embodying the present invention.

FIG. 4 is a plane view of a 2-electrode system of an immunobiosensor embodying the present invention.

FIG. 5 is a structural view of matrix part of an immunobiosensor embodying the present invention.

FIG. 6 is a perspective view of an immunobiosensor embodying the present invention.

FIG. 7 (A) - (F) illustrates the principle of generating a signal in the immunobiosensor embodying the present invention.

FIG. 8 is a perspective view of the immunobiosensor for measuring surface antibody and surface antigen of Hepatitis B virus concurrently according to one embodiment of the present invention.

FIG. 9 is a perspective view of a multi-immunobiosensor for measuring multi-components according to another embodiment of the present invention.

### DETAILED DESCRIPTION OF PREFERRED EMBODIMENT

An embodiment of the present invention is described by referring to the figures attached.

In a currently preferred embodiment the electrochemical immunobiosensor is composed of an electrode part and a chromatography (or capillary) matrix, wherein the electrode part is formed on an insulating base plate using thick-film technology, and an immunochemical reaction occurs by developing the sample by chromatography or capillary action when sample is applied.

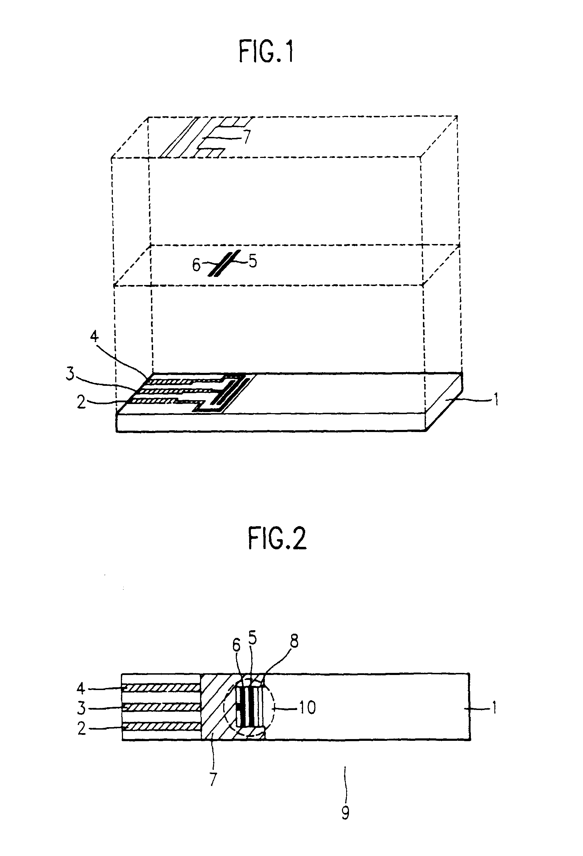

The structure of an electrochemical immunobiosensor is explained with reference to FIGs. 1 to 6.

Electrodes, of the type in a known sensor using an electrochemical principle, have a 2-electrode system composed of a reference electrode and a working electrode, or a 3-electrode system wherein a counter electrode is introduced thereto. Oxidation or reduction of electroactive species occurs on the working electrode under a certain applied potential with respect to the reference electrode.

As shown in FIG.1 and 2, electrode system part(9) consisting of a 3-electrode system is constructed of a conduction path(2,3,4) printed with silver(Ag) paste, a working electrode(5) printed with carbon paste, a counter electrode(6), an Ag/AgCl reference electrode(8) and an insulating layer(7) formed on an insulating base plate(1) made of a polymer such as polyester, polyvinyl chloride or polycarbonate.

FIG 2. is a plane view of an electrode system part(9) which is constructed on an insulating base plate.

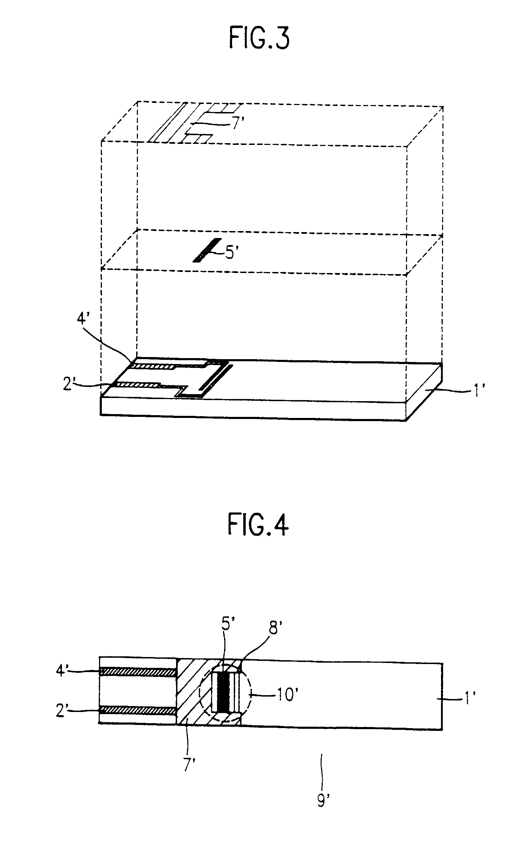

On the other hand, an electrode system consisting of 2-electrode system comprises only a working electrode(5') and a reference electrode(8') as shown in FIGs. 3 and 4.

In general, the material for preparing the working electrode includes carbon, platinum, gold, ruthenium dioxide, or the like.

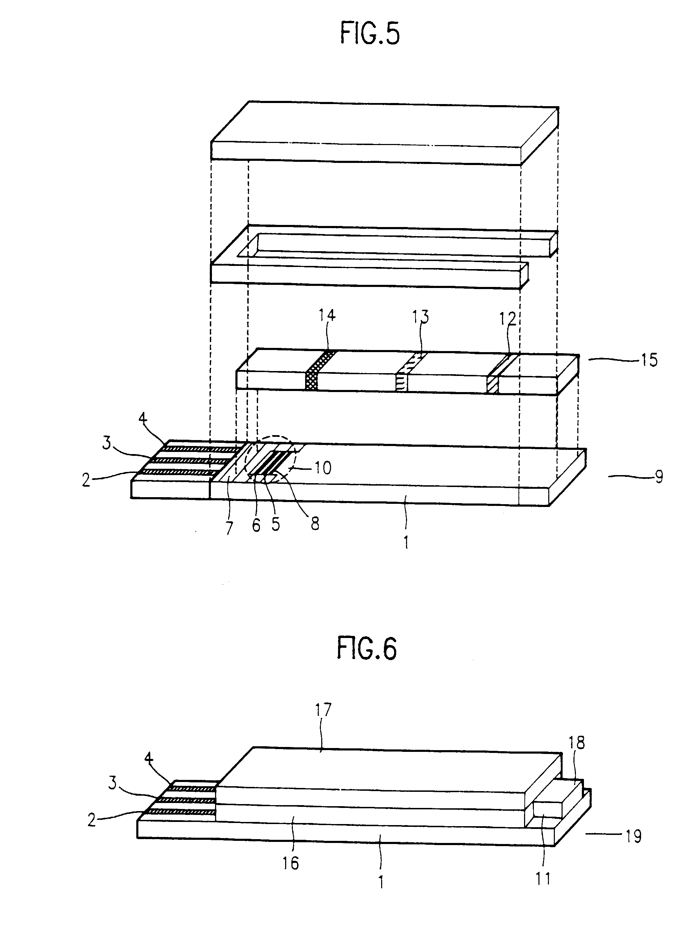

The materials for constructing the matrix part(15) which generates an immunochemical reaction include filter paper, nitrocellulose, polystyrene, polyvinyl, activated nylon film, or the like, as illustrated in FIG. 5. On the right upper side of the matrix part(15), i.e. assay the starting point, an analyte-cytolytic agent conjugate is absorbed, while on the central upper side, i.e. competition point(13), an antibody(or antigen) which can bind to the analyte is strongly bound so that it cannot move, and on the left upper side, i.e. signal-generating point, are immobilized liposomes containing electroactive species such as ferrocene derivatives (e, g. ferrocene, dimethylferrocene), potassium ferrocyanide, viologen derivatives, 2,6-dichlorophenolindophenol (DCPIP), p-aminophenol, tetrathiafulvalene (TTF), tetracyanoquino-dimethane (TCNQ), phenazine methosulphate (PMS), Meldola's blue (7-dimethylamino-1,2-benzophenoxazine), 1,2-benzophenoxazine-7-one (BPO), thionin, or the like.

The matrix part(15) has the function of generating an immunochemical reaction as well as providing electrolytes in the sample to the electrode part(10) by mobilizing the analyte-cytolytic agent conjugates absorbed on the matrix.

As shown in FIG. 5, the matrix part(15) and electrode system(9) having characteristics mentioned above are integrally combined to the electrode portion(10) exposed by an insulating layer on the base electrode, and the portion(14) to which liposomes containing electroactive species are immobilized on the matrix part(15), to complete the immunobiosensor (19).

FIG. 6 is a perspective view of the immunobiosensor completed by the processes mentioned above, and symbol 18 shows sample application part.

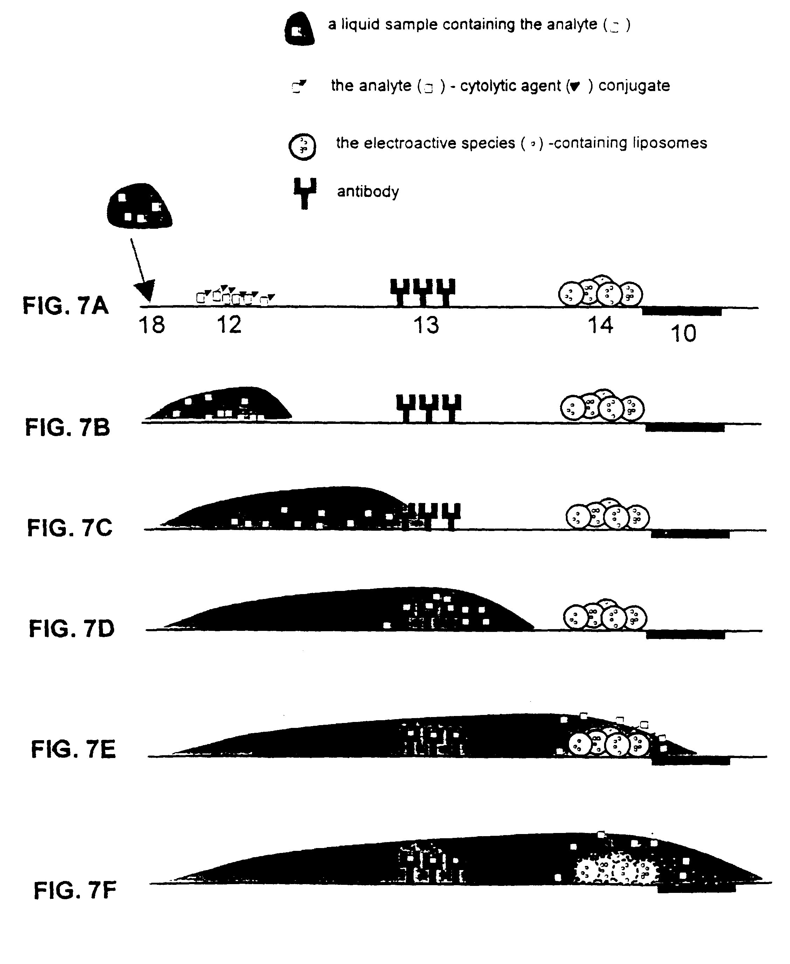

The principles of generating signals by the immunobiosensor are explained with reference to FIG. 7.

In the case where the analyte is an antigen, a predetermined amount of a liquid sample containing the analyte is dropped on the sample application part(18) of the sensor [FIG. 7]. Then, the sample is mixed with the analyte-cytolytic agent conjugates absorbed on the assay starting point(12) of the matrix part(15) as shown in FIG.7, and starts to move with the analyte in the sample by using the liquid in the sample as a carrier, toward the electrode by chromatographic principle [FIG. 7(C)]. In the competition point (13) to be reached at this time, an antibody which specifically binds to the analyte is immobilized on the matrix. To occupy the binding site for antigen-antibody, the analyte and analyte-cytolytic agent conjugates in the sample compete each other [FIG.7(D)].

Thus, if a large amount of the analyte is present in the sample, a lot of the analyte-cytolytic agent passes through the competition point(13) to reach the liposomes on the signal-generating point(14) just over the electrode, to cause the liposome lysis. [Fig.7(E)]. As a result of the immunochemical reaction, electroactive species in the liposomes are released proportional to the concentration of the analyte present in the sample solution, and the released electroactive species diffuse and reach the electrode part(10) [FIG.7(F)]. As the current is generated between the working electrode(5) and the counter electrode(6) upon oxidizing or reducing the electroactive species on the working electrode applied with a certain potential compared to the reference electrode, a current value proportional to the concentration of the analyte present in the sample solution can be obtained.

On the contrary, if the analyte is an antibody, an antigen which specifically binds with the analyte is immobilized on the competition point(13) on the matrix in FIG. 7, so that an assay of the analyte (antibody) can be performed by the same principle.

As described above, the embodiments of the invention may be applied in all immunoassays using the affinity between ligand and receptor such as conventional antigen-antibody reaction [e.g. are biotine-avidin, immunoglobulin G-protein A, hormone-hormone receptor, DNA-DNA, RNA-RNA and drug-drug receptor].

Here-in-after, an immunobiosensor which allows a measure of viruses such as Hepatitis virus and AIDS virus, pathogenic microbes such as *Salmonella* and *Legionella,* proteins such as immunoglobulin, lipoprotein such as HDL and LDL, hormones such as human growth hormone and TSH(Thyroid Stimulating Hormone), antibiotics such as gentamicine and tobramicine, and drugs such as digoxin and theophylline and a multi-immunobiosensor which enables measurement of multi-concurrently will be described in more detail by reference to the following examples.

### Example 1 : Immunobiosensor for measuring surface antigen of Hepatitis B virus(HBsAg)

Surface antigen of Hepatitis B virus(HBsAg)is is a marker for determination whether A person has been infected by Hepatitis B virus or not.

At first, the fabrication for base electrode is carried out in the following order:

As in FIG. 1 and 2, a polyester insulating base plate(1) with 0.3 mm thickness is washed with aceton and silver(Ag) paste is printed thereon and conduction path(2,3,4) and connection paths are formed by heating at 110 °C for 10 minutes. Carbon paste is printed on a working electrode(5) and counter electrode(6) portions and heat-treated as above. And after insulating layer(7) is formed by printing insulating paste thereon, electrode portion(10) exposed by insulating layer is treated with 100 mM FeCl₃ solution for 1 minutes and remaining FeCl₃ solution is washed off by distilled water to form a Ag/AgCl reference electrode(8). Thus, electrode system composed of a working electrode(5), a counter electrode(6) and a reference electrode(8) has been completed.

The fabrication of electroactive species-containing liposome is carried out in the following order:

The electroactive species-containing liposomes are prepared in the form of LUV(Large Unilamella Vesicle) or MLV(Multilamella Lipid Vesicle) by general methods. Namely, phospholipid such as DPPC(dipalmitoylphosphatidylcholine), DPPG(dipalmitoylphosphatidyl glycerol) and cholesterol is dissolved in proper ratio in an organic solvent such as chloroform and the solution is placed in a glass vessel for evaporation under reduced pressure to form a lipid film. A solution of 100 mM potassium ferrocyanide dissolved in phosphate buffer (pH 7.4) is added thereto and the mixed solution is stirred vigorously to form an opaque liposome phase with particle sizes of 100-1000 nm.

A chromatographic matrix is fabricated as follows:

A paper for chromatography such as cellulose paper is cut into 6 mm X 50 mm size and matrices are pretreated by dipping into 0.1 M sodium carbonate buffer wherein 2.5 g of casein is dissolved, and washed with distilled water repeatedly and then dried. As in FIG. 5, potassium ferrocyanide-containing liposome is immobilized on a signal generating point(14) in pretreated matrix part(15) and an antibody binding with target antigen(HBsAg) is immobilized on a competition point(13). A conjugate wherein a cytolytic agent, melittin, is bound to the antigen(HBsAg) is absorbed to the assay starting point(12).

As shown in FIG. 5, matrix part(15) and the electrode system(9) fabricated as above are combined together so that the electrode portion(10) exposed by the insulating layer on base electrode is in accord with the portion(14) wherein the liposome of matrix part(15) is immobilized, using doubled sided adhesive tape (16) and a polyester upper supporting layer(17) to complete the immunobiosensor(19) for measuring surface antigen of Hepatitis B virus.

At measurement, sample blood is dropped on a sample application part(18) and melittin-HBsAg conjugate, absorbed at the assay starting point(12) of the matrix part(15), begins to move with the sample by developing through the matrix. Upon approaching the competition point(13), a surface antigen(HBsAg), measured species in the sample, competes with a melittin-HBsAg conjugate for binding and as a result, melittin, the cytolytic moiety of melittin-HBsAg conjugate reaching the signal generating point(14) through the competition point(13), ruptures the liposome and potassium ferrocyanide contained in liposome is released. At this time, on the 200 mV applied working electrode(5) compared to the reference electrode(8), potassium ferrocyanide is oxidized (Fe(CN)₆⁻⁴ ---> Fe(CN)₆₋³ + e⁻) and the current is resulted in proportion to HBsAg concentration sample.

### Example 2 : Immunobiosensor for measuring surface antibody of Hepatitis B Virus(anti-HBsAg)

Surface antibody of Hepatitis B virus is a marker for determination whether antibody for Hepatitis B virus, in other words, immunity has been formed or not.

The fabrications of base electrode and the preparation of electroactive species-containing liposome are carried out in accordance with the same procedure as in Example 1.

The fabrication of matrix for immunochemical reaction is carried out in the following order:

A paper for chromatography such as cellulose paper is cut into 6 mm X 50 mm size and matrices are pretreated by dipping into 0.1 M sodium carbonate buffer wherein 2.5 g of casein is dissolved, and washed with distilled water repeatedly and then dried. The potassium ferrocyanide-containing liposome is immobilized on a signal generating point(14) in pretreated matrix part(15) and an antigen binding with target antibody is immobilized on a competition point(13). A conjugate comprising a cytolytic agent, melittin, bound to the antibody, is absorbed to the assay starting point(12).

As in FIG. 5, chromatographic matrix part(15) and the electrode system(9) fabricated as above are combined together so that electrode portion(10) exposed by the insulating layer on base electrode is in accord with the portion(14) wherein liposomes of matrix part(15) are immobilized, using double-sided adhesive tape(16) and a polyester upper supporting layer(17) to complete the immunobiosensor(19) for measuring surface antibody of Hepatitis B virus.

At measurement, a sample of blood is dropped on the sample application part(18) and melittin-surface antibody conjugate absorbed at the assay starting point(12) of the matrix part(15) begins to move with the sample by developing through the matrix. Upon approaching the competition point(13), surface antibody(anti-HBsAg), the measured species in sample, competes with melittin-surface antibody conjugate for binding and as a result, melittin, cytolytic moiety of melittin-surface antibody conjugate reaching the signal generating point(14) through the competition point(13), ruptures the liposomes and potassium ferrocyanide contained in the liposomes is released. At this time, on the 200 mV applied working electrode(5) compared to the reference electrode (8), potassium ferrocyanide is oxidized(Fe(CN)₆⁻⁴ ---> Fe(CN)₆⁻³ + e⁻) and the resulting current is in proportion to surface antibody(anti-HBsAg) concentration in sample.

### Example 3 : Immunobiosensor for measuring theophylline

The fabrication of base electrode is carried out in accordance with the same procedure as in Example 1. The fabrication of electroactive species-containing liposome is carried out in the following order:

The electroactive species-containing liposome is prepared in forms of LUV(Large Unilamella Vesicle) or MLV(Multilamella Lipid Vesicle) by general methods. Namely, phospholipid such as DMPC (dimyristoylphosphatidylcholine) and cholesterol are dissolved in proper ratio in an organic solvent, such as chloroform, and the solution is placed in a glass vessel for evaporation under reduced pressure to form a lipid film. A solution of 100 mM ferrocene dissolved in phosphate buffer (pH 7.4) is added thereto and the mixed solution is stirred vigorously to form an opaque liposome phase with particle sizes of 100 - 1000 nm.

The ferrocene-containing liposome is immobilized on a signal generating point(14) in a pretreated nitrocellulose matrix part(15) and an antibody binding with target theophylline(anti-theophylline) is immobilized on the competition point(13). A conjugate having a cytolytic agent, phospholipase C, bound to theophylline is absorbed to the assay starting point(12).

As in FIG. 5, the matrix part(15) and the electrode system(9) fabricated as above are combined together so that electrode portion(10) exposed by the insulating layer on base electrode is in accord with the portion(14) wherein liposomes of matrix part(15) are immobilized, using double-sided adhesive tape(16) and a polyester upper supporting layer(17) to complete the immunobiosensor(19) for measuring theophylline.

At measurement, a sample of blood is dropped on the sample application part(8) and then, the sample and phospholipase C-theophylline conjugate begin to move by developing through the matrix. Upon approaching the competition point(13), theophylline, the analyte, competes with phospholipase C-theophylline conjugate for binding and as a result, phospholipase C, cytolytic moiety of phospholipase C-theophilline conjugate reaching at the signal generating point(14) through the competition point(13), ruptures the liposomes and ferrocene contained therein will be released. At this time, on the 150 mV applied working electrode(5) compared to reference electrode(8), ferrocene is oxidized(ferrocene --> ferricinium + e⁻) and the resulting current is in proportion to theophilline concentration in sample.

### Example 4 : Immunobiosensor for measuring human growth hormone (HGH)

The fabrication of base electrode is carried out in accordance with the same procedure as in Example 1.

The fabrication of electroactive species-containing liposome is carried out in the following order:

The electroactive species-containing liposome is prepared in the form of LUV(Large Unilamella Vesicle) or MLV(Multilamella Lipid Vesicle) by general methods.

Namely, phospholipid such as DMPC(dimyristoylphosphatidylcholine) and cholesterol are dissolved in proper ratio in an organic solvent such as chloroform and the solution is placed in a glass vessel for evaporation under reduced pressure to form a lipid film. A solution of 100 mM DCPIP(2,6-dichlorophenolindophenol) dissolved in phosphate buffer (pH 7.4) is added thereto and the solution is stirred vigorously to form an opaque liposome phase with particle sizes of 100-1000 nm.

The fabrication of matrix for immunochemical reaction is carried out in the following order:

The DCPIP-containing liposome is immobilized on the signal generating point(14) in pretreated nitrocellulose matrix part(15) and an antibody(anti-HGH) binding with target human growth hormone(HGH) is immobilized on the competition point(13). A conjugate having a cytolytic agent, phospholipase C, bound to human growth hormone is absorbed to the analysis beginning point(12).

As in FIG. 5, the matrix part(15) and the electrode system(9) fabricated as above are combined together so that the electrode portion(l0) exposed by the insulating layer on base electrode is in accord with the portion(14) wherein liposomes of matrix part(15) are immobilized, using double-sided adhesive tape(16) and a polyester upper supporting layer(17) to complete the immunobiosensor(19) for measuring HGH.

At measurement, a sample of blood is dropped on sample application part(18) and then, sample and phospholipase C-HGH conjugate begin to move by developing through the matrix. Upon approaching to the competition point(13), HGH, competes with phospholipase C- HGH conjugate for binding and as a result, phospholipase C, cytolytic moiety of phospholipase C-theophilline conjugate reaching the signal generating point(14) through the competition point(13), ruptures liposomes and DCPIP contained therein will be released. At this time, on the 150 mV applied working electrode(5) compared to the reference electrode(8), DCPIP is reduced and the resulting current is in proportion to HGH concentration in sample.

### Example 5. Immunobiosensor for concurrent measuring surface antibody and surface antigen of Hepatitis B virus

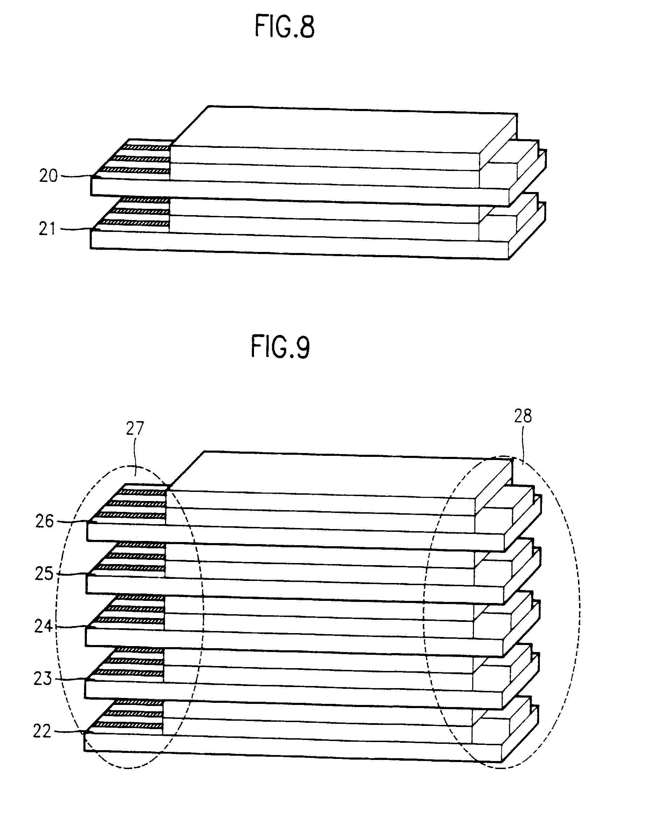

The immunobiosensor for measuring surface antigen of Hepatitis B virus(HBsAg) and the immunobiosensor for measuring surface antibody of Hepatitis B virus(anti-HBsAg) fabricated respectively as in above Example 1 and 2, are bound by overlapping as in FIG. 8 to complete an immunobiosensor for measuring surface antigen and surface antibody of Hepatitis B virus concurrently.

### Example 6. Immunobiosensor for measuring multicomponents

Similar to above Example 5, the individual immunobiosensors (22-25) fabricated respectively as in Example 1 to 4 above, are bound by overlapping as in FIG. 9 to complete a multi-immunobiosensor for measuring multicomponents. At this time, identical electroactive species-containing liposomes are used for individual immunobiosensors (22-25), and control sensor(26) generating a kind of reference signal is overlapped thereon to fabricate the multi-immunobiosensor for measuring multicomponents. Hence, electrode signal output from individual immunobiosensors(22-25) will be amplified differentially from background signal due to no relevance to immunochemical reaction and detection of signal without interference effect can be obtained. In control sensor (26) generating the reference signal, electroactive species-containing liposomes are immobilized only at the signal generating point(14) on nitrocellulose matrix part(15). Therefore, in the case that a cytolytic agent is present in sample or in the case that a liposome is ruptured without immunochemical reaction, accurate measurement will be possible. In sample measurement, measurement circuit connection part(27) is connected to a measurement apparatus and a sample is applied to react the sample contact portion (28) of the multi-immunobiosensor for the measuring multicomponents.

As described above, because an electrochemical immunobiosensor embodying the present invention takes advantages of liposomes wherein electroactive species as a signal-generating material are contained in excess amount and together with the measurement system due to the simple electrochemical principle than any other methods, the described immunobiosensor measures the result of antigen-antibody reaction directly.

According to the immunobiosensor described, all analyses are performed in a short time by simply placing the sample on the specific portion of the sensor and thus, it will be an excellent immunoassay means that can be applied in quick and easy way in various clinics or in the field.

Also because a disposable strip-type immunobiosensor embodying the present invention is packaged and supplied in a dry form, it has an advantage of enhancing the user's convenience.

The disposable strip-type immunobiosensor embodying the present invention provides a method that allows a patient's health condition to be diagnosed directly on the spot in a clinic of a private hospital as well as a general hospital and also greatly serves for national health and welfare due to a rapid prescription.
Therefore, the immunobiosensor embodying the present invention has an advantage that the present product differs from typical analysis systems taking several hours.

The matrix embodying the invention has a structure that can be characterized in that a measuring analyte analyte-cytolytic agent conjugate, an antigen(or an antibody) selectively binding with the analyte and an electroactive species-containing liposome are respectively absorbed or fixed onto a specific portion of a chromatographic matrix phase wherein an immunochemical reaction used in the present invention is carried out. Hence, the described embodiment has an advantage applicable to general ligand-receptor binding, such as immunoglobulin G-protein A, hormone-hormone receptor, DNA-DNA, RNA-RNA and drug-drug receptor as well as typical antigen-antibody reaction.

Therefore the described embodiment has an advantage to fabricate a immunobiosensor which enables measurement of viruses, pathogenic microbes, lipoproteins, hormones, antibiotics and drugs, and a multi-immunobiosensor for measuring multicomponents concurrently.

Meanwhile, because liposome-analyte conjugate can be fabricated according to the embodiment, influence of the invention is very great. Namely, liposome-analyte conjugate instead of said cytolytic agent-analyte conjugate is fabricated and applied with an analyte in matrix, and then as result of immunochemical reaction, liposome-analyte conjugate passing through the competition point is ruptured by the cytolytic agent immobilized around electrode to generate signal.

As described above, immunobiosensor can be practically utilized according to the described embodiment.

## Claims

1. An electrochemical immunobiosensor which comprises an electrode part where a plurality of electrodes are formed on an insulating base plate and a part of the electrode is exposed by selectively forming an insulating layer on the electrode; and a matrix part where a portion absorbed with analyte-cytolytic agent conjugates, a portion with an immobilized receptor which binds the analyte, and a portion fixed with electroactive species-containing liposomes, to perform the functions of liposome-based signal amplification and immunochromatography; and said electrode portion exposed in the electrode part and the liposome portion in the matrix are integrally combined.

2. An electrochemical biosensor according to claim 1, wherein the matrix part is made of a material selected from a group consisting of filter paper of cellulose or nitrocellulose, polystyrene, polyvinyl and activated nylon film.

3. An electrochemical biosensor according to claim 1, wherein the electroactive species-containing liposome is made of phospholipid such as dipalmitoylphosphatidylcholine (DPPC) , dipalmitoylphosphatidylglycerol (DPPG), dimyristoulphosphatidylcholine (DMPC) or cholesterol.

4. An electrochemical biosensor according to claim 3, wherein the electroactive species entrapped in the liposomes are selected from a group consisting of ferrocene derivatives(e.g. ferrocene, dimethylferrocene), potassium ferrocyanide, viologen derivatives, 2,6-dichlorophenolindophenol (DCPIP), p-aminophenol, tetrathiafulvalene (TTF), tetracyanoquino-dimethane (TCNQ), phenazine methosulphate (PMS), Meldola's blue (7-dimethylamino-1,2-benzophenoxazine), 1,2-benzophenoxazine-7-one (BPO) and thionin.

5. An electrochemical biosensor according to claim 1, wherein the receptor and the analyte, comprise a ligand-receptor pair selected from the group consisting of antigen-antibody, biotin-avidin, immunoglobulin G-protein A, hormone-hormone receptor, DNA-DNA, RNA-RNA and drug-drug receptor.

6. An electrochemical biosensor according to claim 1, wherein the cytolytic agent is a physiologically bioactive material such as melittin or phospholipase C.

7. An multi-immunobiosensor for assaying multi-components which multi-immunobiosensor comprises a plurality of overlapping electochemical immunobiosensors which comprise an electrode part where a plurality of electrodes are formed on an insulating base plate and a part of the electrode is exposed by selectively forming an insulating layer on the electrode; and a matrix part where a portion absorbed with analyte-cytolytic agent conjugates, a portion with an immobilized receptor which binds with the analyte, and a portion immobilized with electroactive species-containing liposomes; and said electrode portion exposed in the electrode part and the liposome portion in the matrix are integrally combined, with a control sensor which generates reference signals to enable simultaneous measurement of physiologically important multi-component species.

8. An electrochemical biosensor according to claim 7, wherein the matrix part is made of a material selected from a group consisting of filter paper of cellulose or nitrocellulose, polystyrene, polyvinyl and activated nylon film.

9. An electrochemical biosensor according to claim 7, wherein the electroactive species-containing liposome is made of phospholipid such as dipalmitoylphosphatidylcholine (DPPC) , dipalmitoylphosphatidylglycerol (DPPG), dimyristoulphosphatidylcholine (DMPC) or cholesterol.

10. An electrochemical biosensor according to claim 9, wherein the electroactive species entrapped in the liposomes are selected from a group consisting of ferrocene derivatives(e.g. ferrocene, dimethylferrocene), potassium ferrocyanide, viologen derivatives, 2,6-dichlorophenolindophenol (DCPIP), p-aminophenol, tetrathiafulvalene (TTF), tetracyanoquino-dimethane (TCNQ), phenazine methosulphate (PMS), Meldola's blue (7-dimethylamino-1,2-benzophenoxazine), 1,2-benzophenoxazine-7-one (BPO) and thionin.

11. An electrochemical biosensor according to claim 7, wherein the receptor and the analyte, comprise a ligand-receptor pair selected from the group consisting of antigen-antibody, biotine-avidin, immunoglobulin G-protein A, hormone-hormone receptor, DNA-DNA, RNA-RNA and drug-drug receptor.

12. An electrochemical biosensor according to claim 7, wherein the cytolytic agent is a physiologically bioactive material such as melittin or phospholipase C.

13. An electrochemical biosensor according to claim 7, wherein the same electroactive species-containing liposomes are immobilized on each matrix part of each immonobiosensor.

14. An electrochemical biosensor according to claim 7, wherein the control sensor only includes a matrix part immobilized with eletroactive species-containing liposomes.

## Patentansprüche

1. Elektrochemischer Immunbiosensor, der einen Elektrodenteil, worin mehrere Elektroden auf einer isolierenden Grundplatte gebildet sind und ein Teil der Elektrode durch selektives Bilden einer Isolierschicht auf der Elektrode freigelegt ist; und einen Matrixteil umfaßt, worin ein Abschnitt, an den Analyt-cytolytisches Mittel-Konjugate absorbiert sind, ein Abschnitt mit einem immobilisierten Rezeptor, der den Analyten bindet und ein Abschnitt, an den elektroaktive Spezies enthaltende Liposomen fixiert sind, vorliegen, um die Funktionen einer Signalverstärkung auf Liposombasis und einer Immunchromatographie durchzuführen; und worin der im Elektrodenteil freigelegte Elektrodenabschnitt und der Liposomabschnitt in der Matrix integral kombiniert sind.

2. Elektrochemischer Biosensor nach Anspruch 1, worin der Matrixteil aus einem Material hergestellt ist, ausgewählt aus einer Gruppe, bestehend aus Filterpapier aus Cellulose oder Nitrocellulose, Polystyrol, Polyvinyl und aktivierter Nylonfolie.

3. Elektrochemischer Biosensor nach Anspruch 1, worin das elektroaktive Spezies enthaltende Liposom hergestellt ist aus einem Phospholipid, wie etwa Dipalmitoylphosphatidylcholin (DPPC), Dipalmitoylphosphatidylglycerin (DPPG), Dimyristoylphosphatidylcholin (DMPC) oder Cholesterin.

4. Elektrochemischer Biosensor nach Anspruch 3, worin die elektroaktiven Spezies, die in den Liposomen eingeschlossen sind, ausgewählt sind aus einer Gruppe, bestehend aus Ferrocenderivaten (z.B. Ferrocen, Dimethylferrocen), Kaliumferrocyanid, Viologenderivaten, 2,6-Dichlorphenolindophenol (DCPIP), p-Aminophenol, Tetrathiafulvalen (TTF), Tetracyanochino-dimethan (TCNQ), Phenazinmethosulfat (PMS), Meldolablau (7-Dimethylamino-1,2-benzophenoxazin), 1,2-Benzophenoxazin-7-on (BPO) und Thionin.

5. Elektrochemischer Biosensor nach Anspruch 1, worin der Rezeptor und der Analyt ein Ligand-Rezeptorpaar umfassen, ausgewählt aus der Gruppe, bestehend aus Antigen-Antikörper, Biotin-Avidin, Immunglobulin G-Protein A, Hormon-Hormonrezeptor, DNA-DNA, RNA-RNA und Arzneimittel-Arzneimittelrezeptor.

6. Elektrochemischer Biosensor nach Anspruch 1, worin das cytolytische Mittel ein physiologisch bioaktives Material, wie etwa Melittin oder Phospholipase C ist.

7. Multi-lmmunbiosensor zur Untersuchung von Multikomponenten, welcher Multi-lmmunbiosensor mehrere überlagernde elektrochemische Immunobiosensoren umfaßt, welche einen Elektrodenteil, worin mehrere Elektroden auf einer isolierenden Grundplatte gebildet sind und ein Teil der Elektrode durch selektives Bilden einer Isolierschicht auf der Elektrode freigelegt ist; und ein Matrixteil umfassen, worin ein Abschnitt, an den Analyt-cytolytisches Mittel-Konjugate absorbiert sind, ein Abschnitt mit einem immobilisierten Rezeptor, der mit dem Analyten bindet und ein Abschnitt, an den elektroaktive Spezies enthaltende Liposomen immobilisiert sind, vorliegen; und der im Elektrodenteil freigelegte Elektrodenabschnitt und der Liposomabschnitt in der Matrix integral kombiniert sind, mit einem Kontrollsensor, der Bezugssignale erzeugt, um eine gleichzeitige Messung von physiologisch wichtigen Multikomponentenspezies zu ermöglichen.

8. Elektrochemischer Biosensor nach Anspruch 7, worin der Matrixteil aus einem Material hergestellt ist, ausgewählt aus einer Gruppe bestehend aus Filterpapier aus Cellulose oder Nitrocellulose, Polystyrol, Polyvinyl und aktivierter Nylonfolie.

9. Elektrochemischer Biosensor nach Anspruch 7, worin das elektroaktive Spezies enthaltende Liposom hergestellt ist aus einem Phospholipid, wie etwa Dipalmitoylphosphatidylcholin (DPPC), Dipalmitoylphosphatidylglycerin (DPPG), Dimyristoylphosphatidylcholin (DMPC) oder Cholesterin.

10. Elektrochemischer Biosensor nach Anspruch 9, worin die in den Liposomen eingeschlossenen elektroaktiven Spezies ausgewählt sind aus einer Gruppe, bestehend aus Ferrocenderivaten (z.B. Ferrocen, Dimethylferrocen), Kaliumferrocyanid, Viologenderivaten, 2,6-Dichlorphenolindophenol (DCPIP), p-Aminophenol, Tetrathiafulvalen (TTF), Tetracyanochino-dimethan (TCNQ), Phenazinmethosulfat (PMS), Meldolablau (7-Dimethylamino-1,2-benzophenoxazin), 1,2-Benzophenoxazin-7-on (BPO) und Thionin.

11. Elektrochemischer Biosensor nach Anspruch 7, worin der Rezeptor und der Analyt ein Ligand-Rezeptorpaar umfassen, ausgewählt aus der Gruppe, bestehend aus Antigen-Antikörper, Biotin-Avidin, Immunglobulin G-Protein A, Hormon-Hormonrezeptor, DNA-DNA, RNA-RNA und Arzneimittel-Arzneimittelrezeptor.

12. Elektrochemischer Biosensor nach Anspruch 7, worin das cytolytische Mittel ein physiologisch bioaktives Material, wie etwa Melittin oder Phospholipase C ist.

13. Elektrochemischer Biosensor nach Anspruch 7, worin die gleichen elektroaktive Spezies enthaltenden Liposomen auf jedes Matrixteil jedes Immunbiosensors immobilisiert werden.

14. Elektrochemischer Biosensor nach Anspruch 7, worin der Kontrollsensor nur ein Matrixteil umfaßt, an das elektroaktive Spezies enthaltende Liposomen immobilisiert sind.

## Revendications

1. Immunobiocapteurélectrochimique, qui comprend une partie constituée d'électrodes, dans laquelle une pluralité d'électrodes est formée sur une plaque de base isolante, et une portion de l'électrode est exposée en formant sélectivement une couche isolante sur l'électrode ; une partie servant de matrice comportant une portion avec des conjugués analyte-agent cytolytique absorbés, une portion avec un récepteur immobilisé qui se lie à l'analyte, et une portion comportant à l'état fixé des liposomes contenant des entités électro-actives, pour assurer les fonctions d'amplification de signal et d'immunochromatographie basées sur des liposomes ; et ladite portion d'électrode exposée dans la partie constituée d'électrodes et la portion de liposomes dans la matrice sont combinées de manière intégrée.

2. Biocapteur électrochimique suivant la revendication 1, dans lequel la partie servant de matrice est constituée d'une matière choisie dans le groupe consistant en un papier-filtre formé de cellulose ou de nitrocellulose, un film de polystyrène, un film polyvinylique et un film de Nylon activé.

3. Biocapteur électrochimique suivant la revendication 1, dans lequel le liposome contenant des entités électro-actives est formé d'un phospholipide tel que la dipalmitoylphosphatidylcholine (DPPC), le dipalmitoylphosphatidylglycérol (DPPG), la dimyristoylphosphatidylcholine (DMPC) ou le cholestérol.

4. Biocapteur électrochimique suivant la revendication 3, dans lequel les entités électro-actives piégées dans les liposomes sont choisies dans le groupe consistant en dérivés de ferrocène (par exemple ferrocène, diméthylferrocène), ferrocyanure de potassium, dérivés de viologène, 2,6-dichlorophénolindophénol (DCPIP), p-aminophénol, tétrathiafulvalène (TTF), tétracyanoquinodiméthane (TCNQ), méthosulfate de phénazine (PMS), bleu Meldola (7-diméthylamino-1,2-benzophoxazine), 1,2-benzophénoxazine-7-one (BPO) et thionine.

5. Biocapteur électrochimique suivant la revendication 1, dans lequel le récepteur et l'analyte comprennent une paire ligand-récepteur choisie dans le groupe consistant en les paires antigène-anticorps, biotine-avidine, immunoglobuline G-protéine A, hormone-récepteur d'hormone, ADN-ADN, ARN-ARN et médicament-récepteur de médicament.

6. Biocapteur électrochimique suivant la revendication 1, dans lequel l'agent cytolytique est une substance physiologiquement bioactive telle que la mélittine ou la phospholipase C.

7. Multi-immunobiocapteur pour l'analyse de constituants multiples, multi-immunobiocapteur qui comprend une pluralité d'immunobiocapteurs électrochimiques se chevauchant comprenant une partie constituée d'électrodes dans laquelle une pluralité d'électrodes est formée sur une plaque de base isolante et une portion de l'électrode est exposée en formant sélectivement une couche isolante sur l'électrode ; et une partie servant de matrice comportant une portion avec à l'état absorbé des conjugués analyte-agent cytolytique, une portion avec un récepteur immobilisé qui se lie à l'analyte, et une portion comportant à l'état immobilisé des liposomes contenant des entités électro-actives, ladite portion d'électrode exposée dans la partie constituée d'électrodes et la portion constituée de liposomes dans la matrice étant combinées de manière intégrée, avec un capteur témoin qui engendre des signaux de référence pour permettre la mesure simultanée d'entités multiconstituants physiologiquement importantes.

8. Biocapteur électrochimique suivant la revendication 7, dans lequel la partie servant de matrice est constituée d'une matière choisie dans le groupe consistant en un papier-filtre formé de cellulose ou de nitrocellulose, un film de polystyrène, un film polyvinylique et un film de Nylon activé.

9. Biocapteur électrochimique suivant la revendication 7, dans lequel le liposome contenant des entités électro-actives est formé d'un phospholipide tel que la dipalmitoylphosphatidylcholine (DPPC), le dipalmitoylphosphatidylglycérol (DPPG), la dimyristoylphosphatidylcholine (DMPC) ou le cholestérol.

10. Biocapteur électrochimique suivant la revendication 9, dans lequel les entités électro-actives piégées dans les liposomes sont choisies dans le groupe consitant en dérivés de ferrocène (par exemple ferrocène, diméthylferrocène), ferrocyanure de potassium, dérivés de viologène, 2,6-dichlorophénolindophénol (DCPIP), p-aminophénol, tétrathiafulvalène (TTF), tétracyanoquinodiméthane (TCNQ), méthosulfate de phénazine (PMS), bleu Meldola (7-diméthylamino-1,2-benzophénoxazine),1,2-benzophénoxazine-7-one (BPO) et thionine.

11. Biocapteur électrochimique suivant la revendication 7, dans lequel le récepteur et l'analyte comprennent une paire ligand-récepteur choisie dans le groupe consistant en paires antigène-anticorps, biotine-avidine, immunoglobuline G-protéine A, hormone-récepteur d'hormone, ADN-ADN, ARN-ARN et médicament-récepteur de médicament.

12. Biocapteur électrochimique suivant la revendication 7, dans lequel l'agent cytolytique est une substance physiologiquement bioactive telle que la mélittine ou la phospholipase C.

13. Biocapteur électrochimique suivant la revendication 7, dans lequel les mêmes liposomes contenant des entités électro-actives sont immobilisés sur chaque partie servant de matrice de chaque immunobiocapteur.

14. Biocapteur électrochimique suivant la revendication 7, dans lequel le capteur témoin comprend seulement une partie servant de matrice comportant à l'état immobilisé des liposomes contenant des entités électro-actives.
